# EUROPÄISCHE PATENTSCHRIFT

(11) **EP 0 646 361 B1**
(45) Veröffentlichungstag und Bekanntmachung des Hinweises auf die Patenterteilung: **06.08.1997**
(21) Anmeldenummer: 94107722.4
(22) Anmeldetag: 18.05.1994
(51) Int. Cl.: A61B 17/39

(54) **Elektrochirurgisches Hochfrequenz-Instrument**
Electrosurgical high-frequence instrument
Instrument électrochirurgical à hautes fréquences

(30) Priorität: 05.10.1993 DE 4333983
(43) Veröffentlichungstag der Anmeldung: 05.04.1995
(73) Patentinhaber: DELMA ELEKTRO-UND MEDIZINISCHE APPARATEBAU GESELLSCHAFT mbH, D-78532 Tuttlingen (DE)
(72) Erfinder: Fritzsch, Gernod, Dr.-Ing., D-78532 Tuttlingen (DE)
(74) Vertreter: Dipl.-Phys.Dr. Manitz Dipl.-Ing. Finsterwald Dipl.-Ing. Grämkow Dipl.-Chem.Dr. Heyn Dipl.-Phys. Rotermund Morgan B.Sc.(Phys.)

(56) Entgegenhaltungen:
- DE-A- 3 245 570
- FR-A- 2 700 685
- US-A- 3 858 586
- US-A- 4 161 950

## Beschreibung

Die vorliegende Erfindung betrifft ein elektrochirurgisches HF-Instrument nach dem Oberbegriff der Ansprüche 1 und 3.

Derartige elektrochirurgische HF-Instrumente werden häufig bei laparoskopischen Operationen verwendet, wo es auf eine möglichst schmale Ausbildung von Arbeitsspitze und Schaft ankommt. Durch Änderung der an den Elektroden anliegenden HF-Spannung kann die Betriebsart des HF-Instruments zwischen Koagulieren und Schneiden des behandelten Gewebes umgeschaltet werden.

Bekannte monopolare Instrumente sind in Anlehnung an übliche medizinische Bestecke meist hakenförmig oder spatelförmig ausgebildet, wodurch sie einfach zu bedienen und den meisten Chirurgen in der Handhabung vertraut sind. Die bipolaren Instrumente weisen dagegen meist gegeneinander beweglich gelagerte Elektroden auf, mit denen außer dem Koagulieren oder Schneiden ein Klemmen des Gewebes vorgenommen werden kann.

Da die Verwendung von monopolaren Instrumenten z. B. im Abdomen Sicherheitsrisiken für den Patienten und den Chirurgen mit sich bringt, werden dort von sicherheitsbewußten Chirurgen nur bipolare HF-Instrumente verwendet.

Die existierenden bipolaren elektrochirurgischen Instrumente sind jedoch aufwendig und empfindlich, so daß ihre Verwendung im Vergleich zu den bekannten monopolaren Instrumenten eine besondere Geschicklichkeit des Chirurgen erfordert. Darüber hinaus verschmutzen die meisten bekannten bipolaren Instrumente sehr schnell und sind schlecht zu reinigen.

Weiter ist aus der US 4,161,950, gegen welche die unabhängigen Ansprüche abgegrenzt sind, eine elektrochirurgische Messerklinge aus Isoliermaterial bekannt mit einem Paar von auf den Flachseiten der Klingen angeordneten Elektroden, die an einen Radiofrequenzgenerator angeschlossen werden können.

Aus der DE 32 45 570 A1 ist ein bipolares Koagulationsinstrument bekannt, das in einem Halter eingebettet zwei parallele Elektroden aufweist, die in dem Halter gegen Drehungen und gegen Längsverschiebungen gesichert sind. An den Bearbeitungsspitzen sind die Elektroden des vorbekannten Instrumentes abgebogen, damit diese Spitzen trotz des dahinter befindlichen Halters vom Operateur gut gesehen werden können. Die Ausgestaltung zu einem Mikro-Instrument ist möglich.

Aufgabe der Erfindung ist es, ein bipolares elektrochirurgisches Hochfrequenz-Instrument zum Koagulieren und/oder Schneiden von Gewebe der Eingangs genannten Art zu schaffen, welches einfach aufgebaut und zu bedienen ist, eine hohe Sicherheit für den Patienten und den Chirurgen gewährleistet, leicht zu reinigen und zu sterilisieren ist, besonders geeignet für laparoskopische Untersuchungen sowie universell für mechanisch-chirurgische und/oder elektrochirurgische Eingriffe geeignet ist.

Diese Aufgabe wird durch die Merkmale der unabhängigen Patentansprüche gelöst.

Durch die haken- oder spatelförmige Ausbildung der Arbeitsspitze ist eine universelle mechanische Beaufschlagung von Gewebe, wie sie für Haken und Spatel charakteristisch ist, möglich, wobei jedoch gleichzeitig in einem räumlich eng begrenzten Bereich um die Arbeitsspitze herum auch ein elektrochirurgischer Koagulations- und/oder Schneidvorgang ausgelöst werden kann. Da die Arbeitsspitze weitgehend unabhängig von ihrer Ausbildung als zwei Elektroden und eine Isolierschicht aufweisende Baueinheit so kompakt und schmal wie ein rein mechanisch arbeitender Spatel oder Haken ausgebildet sein kann, eignet sich das erfindungsgemäße Instrument besonders für räumlich beengte Verhältnisse, wie sie z.B. bei laparoskopischen Operationen vorliegen.

Die einheitliche und robuste Ausbildung des HF-Instruments ermöglicht eine einfache Handhabung und leichte Reinigung. Beispielsweise kann bei Anlegen einer für den Schneidvorgang geeigneten HF-Spannung an die Elektroden durch einfaches Hinterhaken das zu behandelnde Gewebe durchtrennt bzw. bei Anlegen einer für die Koagulation geeigneten Spannung durch Aufsetzen der Hakenkrümmung auf das zu behandelnde Gewebe dieses koaguliert werden. Gleichzeitig ist durch den Aufbau als bipolares HF-Instrument die nötige Sicherheit für den Patienten und den Chirurgen gewährleistet.

Nach einer bevorzugten Ausführungsform der Erfindung ist zumindest eine der Elektroden im Bereich des freien Endes der Arbeitsspitze verjüngt ausgebildet und/oder teilweise durch ein Isolationsmittel abgedeckt. Dadurch wird in diesem Bereich der Arbeitsspitze die beim Stromübergang von der Elektrode auf das Gewebe vorhandene Stromdichte erhöht, so daß der verjüngte bzw. abgedeckte Bereich der Arbeitsspitze für den Schneidvorgang besonders gut geeignet ist. Insbesondere kann sich diese Verjüngung etwa von der Mitte der Krümmung des Hakens bis zum freien Ende des Hakens erstrecken, während die Elektroden im übrigen Bereich des Hakens, insbesondere im vom freien Ende des Hakens abgewandt angeordneten, radial außen gelegenen Bereich der Krümmung, eine relativ breite Querschnittsfläche aufweisen. Auf diese Weise kann sowohl die Schneideigenschaft der eine Hälfte des Hakens, beispielsweise beim Hinterhaken von Gewebe, als auch die Koagulationseigenschaft der anderen Hälfte des Hakens noch verbessert werden. Bevorzugt sind beide Elektroden verjüngt bzw. abgedeckt ausgebildet, so daß die Arbeitsspitze eine symmetrische Form besitzt.

Nach einer weiteren bevorzugten Ausführungsform der Erfindung ist die Arbeitsspitze bzw. ein sie tragender Grundkörper lösbar an dem länglichen Schaft befestigt. Dadurch können Arbeitsspitzen mit verschiedenen Formen an ein und demselben Schaft befestigt werden, wodurch die Einsatzfähigkeit eines erfindungsgemäß ausgebildeten Instruments weiter erhöht werden kann. Ein weiterer Vorteil besteht darin, daß die bei der Operation stark verschmutzten Spitzen als Einmalprodukt behandelt werden können. Dadurch verringert sich der Reinigungsaufwand und die Hygiene wird verbessert.

Bevorzugt ist dabei die Arbeitsspitze oder ein sie tragender Grundkörper mit einem oder mehreren Rastelementen an dem Schaft befestigt, wodurch ein einfaches und schnelles Lösen der Arbeitsspitze gewährleistet ist. Es sind jedoch auch Befestigungen durch Verschrauben der Arbeitsspitze direkt oder mittels einer Überwurfmutter mit dem Schaft oder über einen Bajonettanschluß denkbar.

Weitere vorteilhafte Ausführungsformen der Erfindung sind in den Unteransprüchen angegeben.

Nachfolgend wird die Erfindung anhand eines Ausführungsbeispiels unter Bezugnahme auf die Zeichnung erläutert; in dieser zeigt:
- Fig. 1: eine schematische, perspektivische Teilansicht eines erfindungsgemäß ausgebildeten HF-Instruments,
- Fig. 2: eine Seitenansicht des HF-Instruments nach Fig. 1,
- Fig. 3: eine Vorderansicht des HF-Instruments nach Fig. 1,
- Fig. 4: eine Draufsicht des HF-Instruments nach Fig. 1,
- Fig. 5: eine Seiten-Teilansicht einer weiteren Ausführungsform der Erfindung,
- Fig. 6: eine Draufsicht auf die Ausführungsform nach Fig. 5,
- Fig. 7: einen schematischen Längsschnitt durch eine weitere Ausführungsform der Erfindung,
- Fig. 8: einen schematischen Längsschnitt durch eine weitere Ausführungsform der Erfindung.

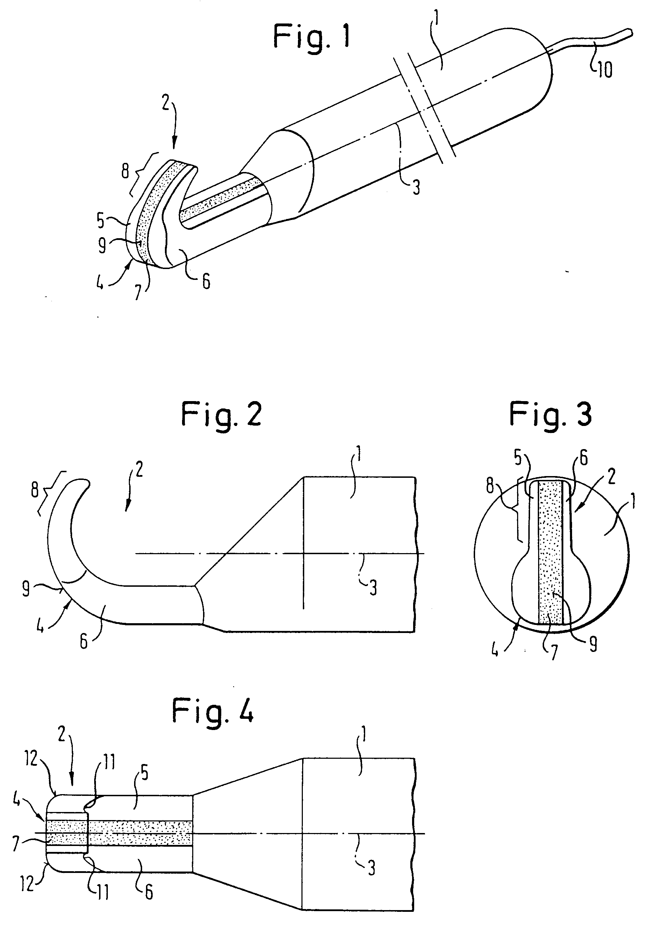

Das in Fig. 1 und 2 dargestellte elektrochirurgische HF-Instrument umfaßt einen Schaft 1, der Stromzuführungen aufnimmt und zugleich als Handhabe dient. Am distalen Ende des Schaftes 1 ist eine Arbeitsspitze 2 angeordnet, deren vorderes Ende als zunächst in Richtung der Längsachse 3 des Schaftes 1 verlaufender und dann nach oben abgebogener Haken 4 ausgebildet ist.

Die Arbeitsspitze 2 besitzt einen schichtförmigen Aufbau, wobei die beiden äußeren Schichten aus Metall bestehen und je eine Elektrode 5, 6 bilden und dazwischen eine Isolationsschicht 7 vorgesehen ist. Die Isolationsschicht 7 füllt den gesamten Zwischenraum zwischen den Elektroden 5, 6 aus, wobei die Übergangsstellen an der Oberfläche der Arbeitsspitze 2 zwischen den Elektroden 5, 6 und der Isolationsschicht 7 kontinuierlich verlaufend ausgebildet sind.

Im Bereich des freien Endes 8 der Arbeitsspitze 2 sind die Elektroden 5, 6 im Vergleich zum Bereich der nach vorne gerichteten Krümmung 9 des Hakens 4 verjüngt ausgebildet.

Am rückwärtigen Ende des Schaftes 1 ist ein Kabel 10 vorgesehen, über das das HF-Instrument mit einem nicht dargestellten elektrochirurgischen Hochfrequenzgenerator verbunden ist.

In der Vorderansicht nach Fig. 3 ist die allseits verjüngte Ausbildung der Elektroden 5, 6 im Bereich des freien Endes 8 der Arbeitsspitze 4 zu erkennen, die sich etwa von der Mitte der Krümmung 9 des Hakens 4 bis zum freien Ende 8 der Arbeitsspitze 2 erstreckt. Im restlichen Bereich der Arbeitsspitze 2 sind die Elektroden 5, 6 so breit ausgebildet, daß ein annähernd kreisförmiger Querschnitt der Arbeitsspitze 2 gegeben ist. Wesentlich ist, daß der Haken 4 so ausgebildet und angeordnet ist, daß er seitlich nirgends über den Schaft 1 hinausragt.

Fig. 4 zeigt die symmetrische Anordnung der Arbeitsspitze 2 und damit der Elektroden 5, 6 bezüglich der Längsachse 3 des Schaftes 1. Die Arbeitsspitze 2 kann aber auch seitlich versetzt zur Längsachse 3 angebracht sein, falls dies beispielsweise zu einer besseren Bedienbarkeit des Instruments oder zu einer besseren Übersichtlichkeit der Operationsstelle führt. Im Gegensatz zu den radial innenliegenden Kanten 11 sind die Kanten 12 im außenliegenden Bereich des Hakens 4 abgerundet ausgebildet.

Die Funktionsweise des elektrochirurgischen HF-Instruments nach den Fig. 1 bis 4 wird nachfolgend näher beschrieben:

Dem durch ein Trokar in das Körperinnere eingeführten elektrochirurgischen HF-Instrument wird je nach Bedarf über das Kabel 10 eine zur Koagulation oder zum Schneiden geeignete HF-Spannung zugeführt. Das Einschalten der HF-Spannung kann dabei beispielsweise über einen nicht dargestellten Schalter, insbesondere einen Fingertaste am Schaft 1, einen Fußschalter, beispielsweise ein Pedal oder direkt am Hochfrequenzgenerator durchgeführt werden.

Zur Koagulation wird auf das zu koagulierende Gewebe bevorzugt der nach vorne weisende Bereich der Krümmung 9 des Hakens 4 aufgesetzt, so daß die in diesem Bereich relativ breit ausgebildeten Elektroden 5, 6 mit dem Gewebe in Berührung kommen. Durch die kontinuierlich verlaufenden Übergänge im Oberflächenbereich der Arbeitsspitze 2 zwischen den Elektroden 5, 6 und der Isolationsschicht 7 sowie durch die abgerundeten Kanten 12 ist ein einwandfreies Gleiten auf der Gewebeoberfläche möglich, so daß Verletzungen durch Verschieben des Instruments entlang des Gewebes ebenso vermieden werden wie Verhakungen der Arbeitsspitze 2 am Gewebe.

Zum Schneiden wird bevorzugt der Haken 4 hinter das zu durchtrennende Gewebe geführt, so daß dieses an der Innenseite des Hakens 4 im Bereich der verjüngt ausgebildeten Elektroden 5, 6 anliegt. Aufgrund der höheren Stromdichte beim Stromübergang von den Elektroden 5, 6 auf das Gewebe im Bereich der verjüngt ausgebildeten Elektroden 5, 6 werden die Gewebezellen aufgesprengt, so daß das Gewebe durch einfaches Zurückziehen des Instruments geschnitten wird.

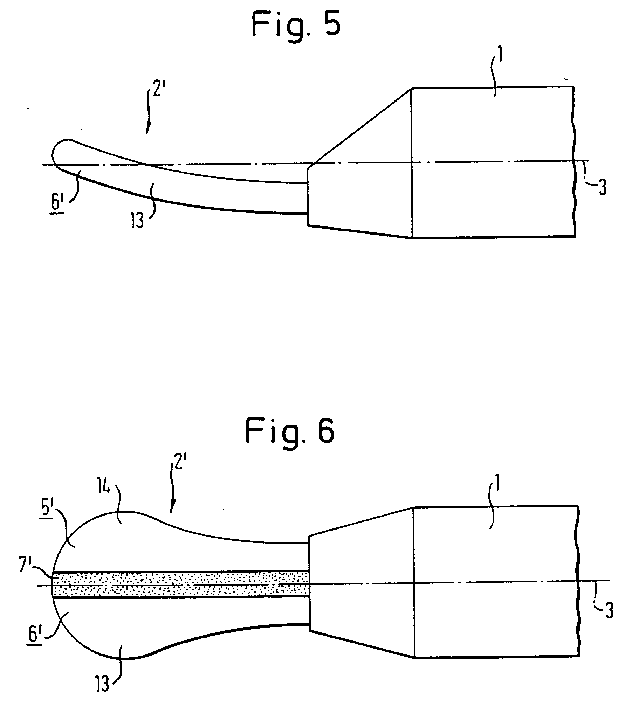

Die Fig. 5 und 6 zeigen ein erfindungsgemäß ausgebildetes elektrochirurgisches HF-Instrument mit einer spatelförmigen, nur leicht gebogenen Arbeitsspitze 2'. Während die in Fig. 6 dargestellte Breite der Isolationsschicht 7' im wesentlichen der Breite der hakenförmigen Arbeitsspitze 2 entspricht, weisen die Elektroden 5', 6' jeweils in ihrem vorderen Bereich eine deutlich ausgeprägte Verdickung 13, 14 auf.

Durch die spatelförmige Ausbildung der Arbeitsspitze 2' ist eine besonders gute großflächige Koagulation von Gewebe möglich, wobei die runden Konturen der Arbeitsspitze 2' eine schonende Behandlung des Gewebes gewährleisten.

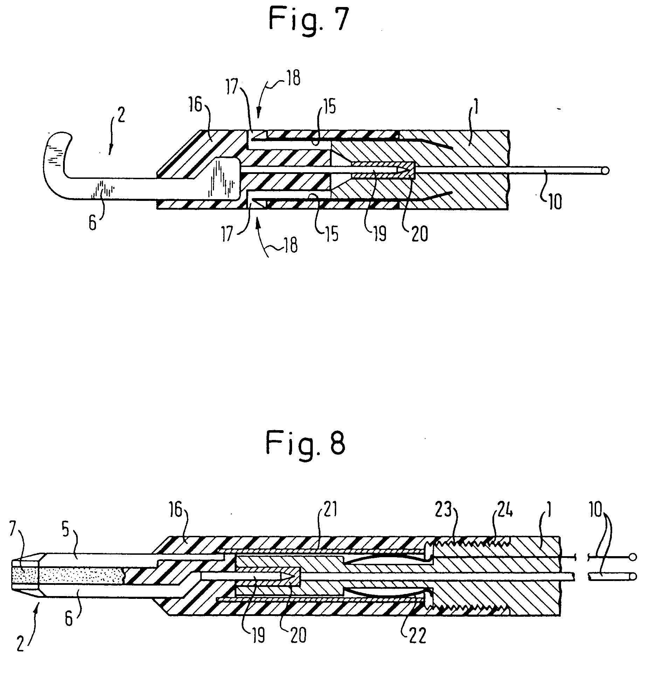

In dem in Fig. 7 dargestellten Längsschnitt durch ein erfindungsgemäß ausgebildetes elektrochirurgische HF-Instrument ist die Arbeitsspitze 2 über am Schaft 1 vorgesehene Rastelemente 15 mit diesem verbunden. Die Arbeitsspitze 2 ist in einem isolierenden Grundkörper 16 befestigt, der Rastausnehmungen 17 aufweist, in welche die Rastelemente 15 eingreifen. Durch Eindrücken der Rastelemente 15 in Richtung der Pfeile 18 ist der Grundkörper 16 und damit die Arbeitsspitze 2 von dem Schaft 1 entkoppelbar.

Die Arbeitsspitze 2 weist weiter an ihrem dem Schaft 1 zugewandten Ende zwei Kontaktstifte 19 auf, von denen aufgrund der Schnittdarstellung in Fig. 7 nur einer gezeigt ist. Die Kontaktstifte 19 sind jeweils mit der Elektrode 5 bzw. 6 verbunden und stellen bei zusammengebautem Instrument über im Schaft 1 vorgesehene Kontaktbuchsen 20 - von denen ebenfalls nur eine dargestellt ist - die elektrische Verbindung zwischen der Arbeitsspitze 2 und dem Kabel 10 und damit die Verbindung zum Hochfrequenzgenerator her. Durch die Steckverbindung der elektrischen Anschlüsse bei gleichzeitiger mechanischer Rastverbindung der Arbeitsspitze 2 bzw. des Grundkörpers 16 mit dem Schaft 1 können verschiedene Arbeitsspitzen 2 sehr einfach und schnell, auch während einer Operation, gegeneinander ausgetauscht werden.

Die elektrische Verbindung kann aber beispielsweise auch direkt durch metallisch ausgebildete Rastelemente hergestellt werden, die zur Oberfläche des Schaftes 1 hin isoliert ausgebildet sind. Auf diese Weise fallen die elektrische und die mechanische Verbindung durch die Doppelfunktion der Rastelemente zusammen.

Fig. 8 zeigt eine weitere Ausführungsform der Erfindung, bei der die Arbeitsspitze 2 über den Grundkörper 16 mit dem Schaft 1 verschraubt ist. Dazu besitzt der Grundkörper 16 an seinem dem Schaft 1 zugewandten Ende ein Innengewinde 23, das mit einem am Schaft 1 vorgesehenen Außengewinde 24 verschraubbar ist.

Die elektrische Verbindung der einen Elektrode 6 zum Hochfrequenzgenerator wird wie nach Fig. 7 über einen in einer Kontaktbuchse 20 angeordneten Kontaktstift 19 hergestellt. Die andere Elektrode 5 ist mit einem Kontaktrohr 21 verbunden, das koaxial zur Längsachse 3 des Schaftes 1 angeordnet ist. Innerhalb des Kontaktrohres 21 sind Schleifkontakte 22 angeordnet, die an der Innenseite des Kontaktrohres 21 angreifen und damit die elektrische Verbindung zwischen den Elektroden 5, 6 der Arbeitsspitze 2 und dem Hochfrequenzgenerator herstellen. Durch die Ausbildung der elektrischen Verbindung mittels der Schleifkontakte 22 innerhalb des koaxial angeordneten Kontaktrohres 21 wird ein unproblematisches Zusammen- bzw. Auseinanderschrauben des Grundkörpers 16 und des Schaftes 1 gewährleistet.

Neben den beschriebenen Verbindungsarten sind zur Verbindung der Arbeitsspitze 2 mit dem Schaft 1 beliebige geeignete lösbare Verbindungsmittel wie beispielsweise Überwurfmuttern, Bajonettverbindungen, Steckverbindungen oder sonstige Schnappverbindungen denkbar.

## Patentansprüche

1. Elektrochirurgisches HF-Instrument zum Koagulieren und/oder Schneiden von Gewebe mit einem einen HF-Spannungsanschluß (10) aufweisenden Schaft (1), an dessen distalem Ende eine Arbeitsspitze (2) vorgesehen ist, bestehend aus einer flachen länglichen Isolierschicht (7) mit zwei gleichen parallen ebenen Oberflächen in einem relativ kleinen Abstand, die sich vom Schaft weg erstrecken, und zwei Elektroden (5; 6), wovon jede eine ebene Oberfläche aufweist, welche parallel zu der einen bzw. der anderen Oberfläche der Isolierschicht liegen, dieselbe Größe wie die eine bzw. andere Oberfläche der Isolierschicht aufweisen und daran so angebracht sind, daß der Übergang von den Oberflächen der Elektroden zu jenen der Isolierschicht kontinuierlich verläuft,
dadurch **gekennzeichnet,**
daß die Einheit aus Isolierschicht (7) und den zwei Elektroden (5, 6) als Haken (4) mit senkrecht zu den parallelen ebenen Oberflächen der Isolierschicht verlaufenden Krümmungsachsen geformt ist.

2. Elektrochirurgisches HF-Instrument nach Anspruch 1,
dadurch **gekennzeichnet,**
daß die Krümmung des Hakens (4) mehr als 90° beträgt.

3. Elektrochirurgisches HF-Instrument zum Koagulieren und/oder Schneiden von Gewebe mit einem einen HF-Spannungsanschluß (10) aufweisenden Schaft (1), an dessen distalem Ende eine Arbeitsspitze (2') vorgesehen ist, bestehend aus einer flachen länglichen Isolierschicht (7') mit zwei gleichen parallen ebenen Oberflächen in einem relativ kleinen Abstand, die sich vom Schaft weg erstrecken, und zwei Elektroden (5'; 6'), wovon jede eine ebene Oberfläche aufweist, welche parallel zu der einen bzw. der anderen Oberfläche der Isolierschicht liegen, dieselbe Größe wie die eine bzw. andere Oberfläche der Isolierschicht aufweisen und daran so angebracht sind, daß der Übergang von den Oberflächen der Elektroden zu jenen der Isolierschicht kontinuierlich verläuft,
dadurch **gekennzeichnet**,
daß die Isolierschicht (7') als Stab mit rechteckigem Querschnitt gebildet ist und die Elektroden (5', 6') eine symmetrische Verdickung (13, 14) mit einem abgerundeten lateralen Ansatz nahe dem distalen Ende derart aufweisen, daß die Einheit aus Isolierschicht (7') und den zwei Elektroden (5', 6') die Gestalt eines Spatels aufweist.

4. Elektrochirurgisches HF-Instrument nach Anspruch 3,
dadurch **gekennzeichnet,**
daß die Elektroden (5', 6') beidseits der Isolierschicht sich von dieser zumindest stellenweise um einen Abstand erstrecken, der größer als die Breite der Isolationsschicht (7') ist.

5. Elektrochirurgisches HF-Instrument nach einem der vorhergehenden Ansprüche,
dadurch **gekennzeichnet,**
daß zumindest eine der Elektroden (5, 6) im Bereich des freien Endes (8) der Arbeitsspitze verjüngt ausgebildet und/oder teilweise durch ein Isolationsmittel abgedeckt ist.

6. Elektrochirurgisches HF-Instrument nach einem der vorhergehenden Ansprüche,
dadurch **gekennzeichnet,**
daß die Arbeitsspitze (2, 2') bzw. ein sie tragender Grundkörper (16) lösbar an dem Schaft (1) befestigt ist.

7. Elektrochirurgisches HF-Instrument nach Anspruch 6,
dadurch **gekennzeichnet,**
daß die Arbeitsspitze (2, 2') bzw. ein sie tragender Grundkörper (16) mit einem oder mehreren Rastelementen (15) an dem Schaft (1) befestigt ist.

8. Elektrochirurgisches HF-Instrument nach Anspruch 6 oder Anspruch 7, bei dem der Hochfrequenzspannungsanschluß (10) sich am distalen Ende des Schaftes (1) befindet,
dadurch **gekennzeichnet,**
daß an der Trennstelle zwischen der Arbeitsspitze (2) bzw. dem sie tragenden Grundkörper (16) und dem Schaft (1) Hochfrequenzspannungssteck- oder -schleifkontakte (19, 20, 21, 22) vorgesehen sind.

9. Elektrochirurgisches HF-Instrument nach Anspruch 1 oder Anspruch 2,
dadurch **gekennzeichnet,**
daß die äußeren Oberflächen der Elektroden (5, 6) teilweise zumindest im wesentlichen parallel zu der vorzugsweise ebenen Isolationsschicht (7) verlaufen.

## Claims

1. Electro-surgical high frequency instrument for the coagulation and/or cutting of tissue comprising a shaft (1) which is provided with a high frequency voltage connection (10) and which has at its distal end a working tip (2) consisting of a flat elongate insulating layer (7) with two similar parallel flat surfaces at a relatively small spacing, which extend away from the shaft, and of two electrodes (5; 6) of which each has a flat surface which lies parallel to the one or other surface of the insulating layer respectively, which have the same size as the one or other surface of the insulating layer respectively and which are so attached to it that the transition from the surfaces of the electrodes to those of the insulating layer extends continuously, characterised in that the unit consisting of the insulating layer (7) and of the two electrodes (5, 6) is formed as a hook (4) with axes of curvature extending perpendicular to the parallel flat surfaces of the insulating layer.

2. Electro-surgical high frequency instrument in accordance with claim 1, characterised in that the curvature of the hook (4) amounts to more than 90°.

3. Electro-surgical high frequency instrument for the coagulation and/or cutting of tissue comprising a shaft (1) which is provided with a high frequency voltage connection (10) and which has at its distal end a working tip (2') consisting of a flat elongate insulating layer (7') with two similar parallel flat surfaces at a relatively small spacing, which extend away from the shaft, and of two electrodes (5'; 6') of which each has a flat surface which lies parallel to the one or other surface of the insulating layer respectively, which have the same size as the one or other surface of the insulating layer respectively and which are so attached to it that the transition from the surfaces of the electrodes to those of the insulating layer extends continuously, characterised in that the insulating layer (7') is formed as a bar of rectangular cross-section and the electrodes (5', 6') have a symmetrical thickened portion (13, 14) with a rounded off lateral bulge close to the distal end in such a way that the unit consisting of the insulating layer (7') and the two electrodes (5', 6') has the shape of a spatula.

4. Electro-surgical high frequency instrument in accordance with claim 3, characterised in that the electrodes (5', 6') extend on both sides of the insulating layer away from the latter at least at some points by a distance which is greater than the width of the insulating layer (7').

5. Electro-surgical high frequency instrument in accordance with one of the preceding claims, characterised in that at least one of the electrodes (5, 6) is of tapered shape in the region of the free end (8) of the working tip and/or is partly covered over by an insulating means.

6. Electro-surgical high frequency instrument in accordance with one of the preceding claims, characterised in that the working tip (2, 2'), or a base body (16) which carries them, is releasably secured to the shaft (1).

7. Electro-surgical high frequency instrument in accordance with claim 6, characterised in that the working tip (2, 2'), or a base body (16) which carries them is secured by one or more latch elements (15) to the shaft (1).

8. Electro-surgical high frequency instrument in accordance with claim 6 or claim 7, in which the high frequency voltage connection (10) is located at the distal end of the shaft (1), characterised in that high frequency voltage plug or slip contacts (19, 20, 21, 22) are provided at the partition point between the working tip (2) or the basic body (16) which carries them and the shaft (1).

9. Electro-surgical high frequency instrument in accordance with claim 1 or claim 2, characterised in that the outer surfaces of the electrodes (5, 6) extend partly at least substantially parallel to the preferably flat insulation layer (7).

## Revendications

1. Instrument électrochirurgical HF pour la coagulation et/ou la coupe de tissus, ayant un manche (1) comportant une connexion de tension HF (10), à l'extrémité distale duquel est prévue une tête opératoire (2) composée d'une couche isolante allongée plate (7) ayant deux surfaces planes parallèles identiques à distance relativement faible , qui s'étendent en s'éloignant du manche, et deux électrodes (5, 6), chacune comportant une surface plane, lesquelles sont placées parallèles à l'une ou l'autre surface de la couche isolante, présentent les mêmes dimensions que l'une ou l'autre surface de la couche isolante et sont disposées attenantes à celles-ci de telle sorte que la transition des surfaces des électrodes à celles de la couche isolante se développe en continu, caractérisé en ce que l'unité composée de la couche isolante (7) et des deux électrodes (5, 6) est conformée en crochet (4) ayant des axes de courbures s'étendant perpendiculairement aux surfaces planes parallèles de la couche isolante.

2. Instrument électrochirurgical HF selon la revendication 1, caractérisé en ce que la courbure du crochet (4) s'élève à plus de 90°.

3. Instrument électrochirurgical HF pour la coagulation et/ou la coupe de tissus, ayant un manche (1) comportant une connexion de tension HF (10), à l'extrémité distale duquel est prévue une tête opératoire (2') composée d'une couche isolante allongée plate (7') ayant deux surfaces planes parallèles identiques à distance relativement faible, qui s'étendent en s'éloignant du manche, et deux électrodes (5', 6'), chacune comportant une surface plane, lesquelles sont placées parallèles à l'une ou l'autre surface de la couche isolante, présentent les mêmes dimensions que l'une ou l'autre surface de la couche isolante et sont disposées attenantes à celles-ci de telle sorte que la transition des surfaces des électrodes à celles de la couche isolante se développe en continu, caractérisé en ce que la couche isolante (7') est formée en barreau avec une section transversale rectangulaire et les électrodes (5', 6') comportent un renflement (13, 14) symétrique avec une saillie latérale arrondie près de l'extrémité distale de telle sorte que l'unité formée de la couche isolante (7') et des deux électrodes (5', 6') présente la forme d'une spatule.

4. Instrument électrochirurgical HF selon la revendication 3, caractérisé en ce que les électrodes (5', 6') s'étendent des deux côtés de la couche isolante, à partir de celle-ci, au moins par endroits d'une distance qui est plus grande que la largeur de la couche isolante (7').

5. Instrument électrochirurgical HF selon l'une des revendications précédentes, caractérisé en ce qu' au moins une des électrodes (5, 6) dans la zone de l'extrémité libre (8) de la tête opératoire est configurée rétrécie et/ou est recouverte partiellement par un moyen isolant.

6. Instrument électrochirurgical HF selon l'une des revendications précédentes, caractérisé en ce que la tête opératoire( 2, 2') ou un corps de base (16) qui la porte est fixé de manière amovible au manche (1).

7. Instrument électrochirurgical HF selon la revendication 6, caractérisé en ce que la tête opératoire (2, 2') ou un corps de base (16) qui la porte est fixé au manche (1) par un ou plusieurs élément(s) d'encliquetage (15).

8. Instrument électrochirurgical HF selon la revendication 6 ou la revendication 7, dans lequel la connexion à la tension haute fréquence (10) se trouve à l'extrémité distale du manche (1), caractérisé en ce qu'à l'endroit de la séparation entre la tête opératoire (2), ou le corps de base (16) qui la porte, et le manche (1) sont disposés des contacts de tension haute fréquence à enfichage ou à balai (19, 20, 21, 22).

9. Instrument électrochirurgical HF selon la revendication 1 ou la revendication 2, caractérisé en ce que les surfaces externes des électrodes (5, 6) s'étendent partiellement au moins essentiellement parallèles à la couche isolante (7), de préférence plane.
